# EUROPEAN PATENT SPECIFICATION

(11) **EP 1 196 101 B1**
(45) Date of publication and mention of the grant of the patent: **07.04.2004**
(21) Application number: 00937929.8
(22) Date of filing: 26.05.2000
(51) Int. Cl.: A61B 17/58, A61B 17/72

(54) **PEDIATRIC INTRAMEDULLARY NAIL**
PÄDIATRISCHER INTRAMEDULLÄRER MARKNAGEL
BROCHE INTRAMEDULAIRE PEDIATRIQUE

(30) Priority: 27.05.1999 US 321369
(43) Date of publication of application: 17.04.2002
(73) Proprietor: Phillips, Jonathan, Orlando, FL 32835 (US)
(72) Inventor: Phillips, Jonathan, Orlando, FL 32835 (US)
(74) Representative: Allman, Peter John
(86) International application number: PCT/US2000/014840
(87) International publication number: WO 2000/072767

(56) References cited:
- US-A- 4 034 013
- US-A- 4 862 883
- US-A- 4 913 137
- US-A- 5 019 079
- US-A- 5 034 013
- US-A- 5 035 697
- US-A- 5 041 115
- US-A- 5 433 718
- US-A- 5 490 409

## Description

### BACKGROUND OF THE INVENTION

### Field of the Invention

The present invention relates to devices and methods for repairing bone fractures, and, more particularly, to intramedullary nails and related internal fixation methods especially suitable for repairing long-bone fractures in children.

### Description of Related Art

The use of intramedullary nails in the repair of long-bone fractures, such as in the femur, has been known in the orthopedic field. Exemplary devices include those known as the Rush and Enders and the Kuntschner nails, as well as those disclosed in U.S. Pat. Nos. 5,713,902 to Friedl; 5,697,930 to Itoman et al.; 5,573,536 to Grosse et al.; 5,562,666 to Brumfield; 5,374,235 to Ahrens; 5,312,406 and 5,167,663 to Brumfield; 5,122,141 to Simpson; 5,066,296, 5,041,114, and 4,776,330 to Chapman et al.; 4,976,258 to Richter et al.; 4,875,475 to Comte et al.; 4,846,162 to Moehring; 4,506,662 to Anapliotis; and 4,475,545 to Ender. US 5,034,013 discloses an intramedullary nail having a tubular elongated body that includes a proximal head portion, an intermediate portion and distal end portion.

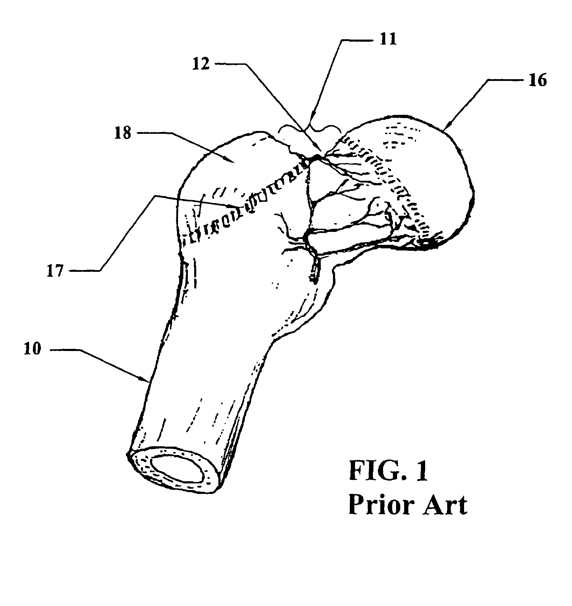

Referring to Fig. 1, a special problem in pediatric orthopedics exists in that reaming through the typical entry point in a femur 10, i.e., the piriformis fossa 11, can be too dangerous for the child. This is due to the presence of an artery 12 that supplies blood to the proximal femur. Specifically, this is the lateral epiphyseal artery 12 which is a branch of the femoral artery. If this artery 12 is damaged during the fixation procedure, such as while the intramedullary canal is being reamed to accept a nail, or possibly during insertion or after insertion of the nail, various complications can result. The lateral epiphyseal artery 12 supplies 75% of the blood to the growing femoral head 16. If this artery 12 is damaged, then much of the femoral head 16 will die or necrose. The femoral head 16 will then heal with an irregular shape which inevitably leads to hip arthritis.
Various nails, such as flexible Rush nails, are non-interlocked meaning that cross fasteners are not used to secure the nail to the bone. These nails are often small diameter rods, on the order of approximately 3-4 mm in diameter. In addition to being flexible to a significant degree prior to plastic deformation, non-interlocked solid nails or rods can be relatively easily bent with plastic deformation to a desired shape. A plurality of these nails or rods are typically driven into the intramedullary canal depending on the support necessitated by the fracture and bone characteristics of the patient. Other more rigid solid or hollow nails are interlocked to the bone using cross fasteners typically at the proximal and distal ends of the nail. Unlike non-interlocked nails, interlocked nails require sufficient cross-sectional dimensions to accommodate holes necessary for the cross fasteners. Currently available interlocked nails can be inserted away from the lateral epiphyseal artery 12 but are so rigid that they migrate during insertion dangerously close to the artery 12 and can endanger it. In addition, the large proximal size of small adult interlocked nails, which have typically been used in children, increases the potential for damage to the growth plate 17 at the proximal femur.

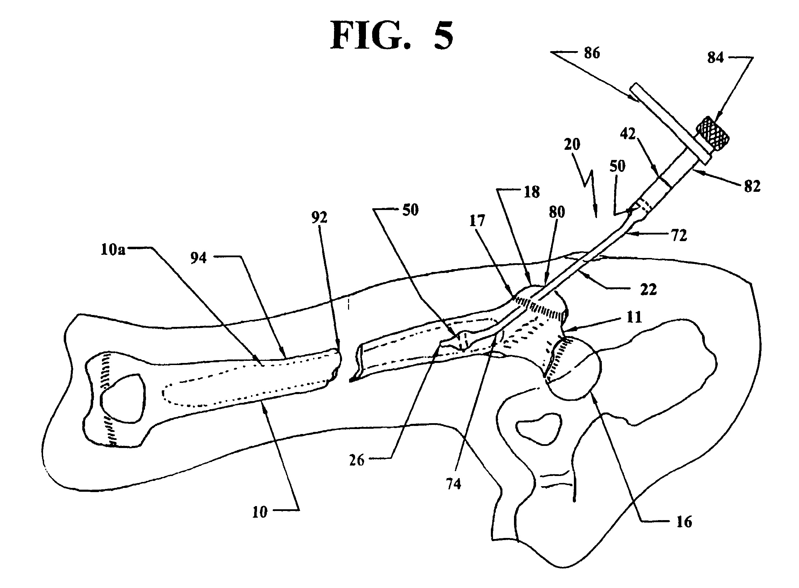

Among possible solutions, retrograde nailing avoids the proximal femur but also has at least one potential problem. The nails must be introduced close to the distal femoral growth plate or physis 19 (Fig. 5) at an awkward angle, potentially causing growth arrest distally on the femur, i.e., adjacent the knee. An approach through the greater trochanter 18 is also well recognized, but usually only one small diameter non-interlocked nail or rod can be used because of the narrow safe entry zone of the greater trochanter. A second small diameter nail or rod needs to be inserted retrograde or through the opposite end of the femur in these situations. These small diameter, flexible nails allow flexure after insertion and the slightly added stress to the bone allowed by this flexure promotes faster bone healing. These non-interlocked nails work well for transverse fractures. However, spiral or comminuted fractures often need additional external support, such as with a cast or brace. This is due to the inability of the non-interlocked nail to effectively prevent rotation or length compromise at the fracture.

It would therefore be desirable to provide an interlocked intramedullary nail, especially suitable for pediatric use, which provides flexibility along a majority of the length of the nail to facilitate faster healing of a fracture, but which also provides for secure interlocking of the nail to the bone with cross fasteners to prevent compromising the fracture due to rotation or shortening at the fracture site. Ideally, such a nail and related methods of insertion would minimize trauma to the growth plates of the femur as well as the arteries that supply blood to the proximal end of the femur while still allowing easy insertion and fixation within the intramedullary canal.

### SUMMARY OF THE INVENTION

In one general aspect, the present invention provides an intramedullary nail for insertion within an intramedullary canal of a long bone and fixing a fracture in the long bone. The nail is especially suitable for adolescent or preadolescent aged children, however, the nail may be useful in other orthopedic applications as well. The nail generally comprises an elongate member having a longitudinal axis, a proximal end section, a distal end section and a solid central section extending between the proximal and distal end sections. The proximal and distal end sections respectively have fastener receiving areas of greater cross sectional dimensions than the central section. The fastener receiving areas each include at least one hole extending transverse to the longitudinal axis of the elongate member for receiving a cross fastener adapted to secure to the bone on opposite sides of the elongate member. The proximal and distal end sections provide rigid anchoring locations relative to the central section and the central section provides flexibility to promote healing of the fracture.

In the preferred embodiment, the central section of the elongate member is curved in the sagital plane to generally follow the curvature of a femur. The proximal and distal end sections are bent out of this plane and form acute angles with respect to the sagital plane. The proximal and distal end sections are each bent laterally to one side of the central section. The side to which the proximal and distal end sections are bent depends on whether the nail will be used in a right or left femur and also allows easier insertions across the fracture. The bend of the distal end section allows for easier insertion of the nail from an insertion point extending through the greater trochanter of the femur. The bend of the proximal end section ensures that the proximal tip is presented directly at the insertion point after fixation so that it may be easily accessed for removal purposes upon healing of the bone.

In general, a method of fixing a fracture in a long bone of a patient, in accordance with the invention, includes providing an elongate member having a solid central section with a cross sectional dimension and having proximal and distal fastener receiving areas of increased cross sectional dimension relative to the cross sectional dimension of the central section. The fastener receiving areas each have at least one hole extending transverse to a longitudinal axis of the elongate member. The method involves inserting the elongate member into the intramedullary canal of the long bone through an insertion point and across the fracture and inserting cross fasteners through each of the holes and into the bone on opposite sides of the elongate member to fix the fracture of the long bone against rotational and lengthwise movements.

The bone nail and method of this invention allow the surgeon to better avoid the critical arterial blood supply to the femoral head which crosses at the piriformis fossa, i.e., the traditional point of entry for an adult nail. Instead, the entry point is on the greater trochanter, a location distinctly lateral of the critical region of the piriformis fossa. The very vulnerable growth plate between the greater trochanter and the femoral neck is also avoided using the nail and entry point in accordance with this invention. Another unique feature of the invention is the ability of the bone nail to be flexible, yet custom bent to match the exact geometry of the proximal femur, while also allowing interlocking with cross fasteners. In general, the preferred inventive nail provides interlockability, a relatively small solid cross section to provide flexure along at least the majority of the length of the nail, and malleability to allow custom bending especially at the proximal end.

The features that characterize the invention, both as to organization and method of operation, together with further objects, features and advantages thereof, will be better understood from the following written description taken in conjunction with the accompanying drawings. It is to be expressly understood that the drawings and detailed description thereof are for the purpose of illustration and description and is not intended as a definition of the limits of the invention. These and other objects attained, and advantages offered, by the present invention will become more fully apparent as the description that now follows is read in conjunction with the accompanying drawings.

### BRIEF DESCRIPTION OF THE DRAWINGS

Fig. 1 is a perspective view of a portion of a femur including the femoral head and the greater and lesser trocantic regions.
Fig. 2 is a side elevational view of an intramedullary nail of the present invention shown in the anterior-posterior plane.
Fig. 3 is an elevational view of the nail shown in Fig. 2, but illustrating proximal and distal bends.
Fig. 4 is an elevational view of the nail shown in Fig. 3, but rotated 90°.
Fig. 5 is a schematic view of the step of initially inserting the bone nail of Figs. 3 and 4 into a femur.
Fig. 6 is a schematic view similar to Fig. 5, but illustrating the nail further inserted across the bone fracture.
Fig. 7 illustrates a drill rig and fastener inserting rig being used to cross fastened the nail after full insertion.
Fig. 8 is a schematic illustration of the fully inserted and cross fastened bone nail.
Fig. 8A is a fragmented, partially sectioned view of the distal end section of the bone nail and the cross fastener of the present invention.
Fig. 9 is an elevational view showing one illustrative bending device being used to bend the proximal end section of the bone nail.

### DETAILED DESCRIPTION

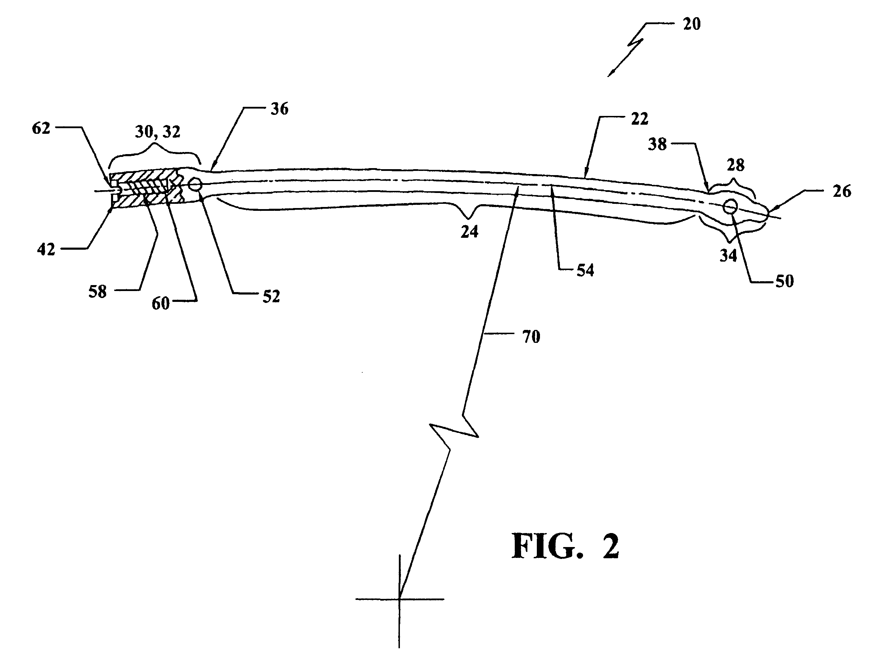

An exemplary intramedullary nail 20, as illustrated in FIGS. 2-4, comprises an elongate member 22. In an embodiment intended for use in a femur of a child or adolescent, typically ages 6-14, for example, the nail 20 is formed from titanium and has a generally cylindrical shape with a diameter along a solid central section 24 of between 4mm and 7mm, although this is not intended as a limitation. Central section 24 should have a certain amount of elastic flexibility to permit accurate placement within the femoral canal as discussed below. Such elastic flexibility, which permits some flexure during movement and weight bearing activity, confers an additional benefit in that it has been found to stimulate bone healing. The nail 20 preferably ends in a rounded, smooth and tapered distal tip 26.

The nail 20 has two widened sections or fastener receiving areas 28, 30 in respective proximal and distal end sections 32, 34. Each fastener receiving area 28, 30 is formed with an increased cross sectional dimension relative to central section 24. Also, smooth, gradual transitions 36, 38 with the central section 24 avoid sharp edges along the length of nail 20. The distal fastener receiving area 28 is positioned adjacent to but in spaced relation from distal tip 26. The distal fastener receiving area 28 tapers on both proximal and distal sides thereof. The proximal fastener receiving area 30 extends to proximal tip 42; that is, there is a distal taper but no proximal taper, and the widened proximal fastener receiving area 30 instead continues to the proximal tip 42 and forms the entire proximal end section 32.

Each fastener receiving area 28, 30 includes a generally cylindrical hole 50, 52 extending generally normal to the portion of the longitudinal axis 54 in which it is located for receiving a fastener (not shown). Holes 50, 52 preferably have diameters in a range of 3 mm to 4½ mm.

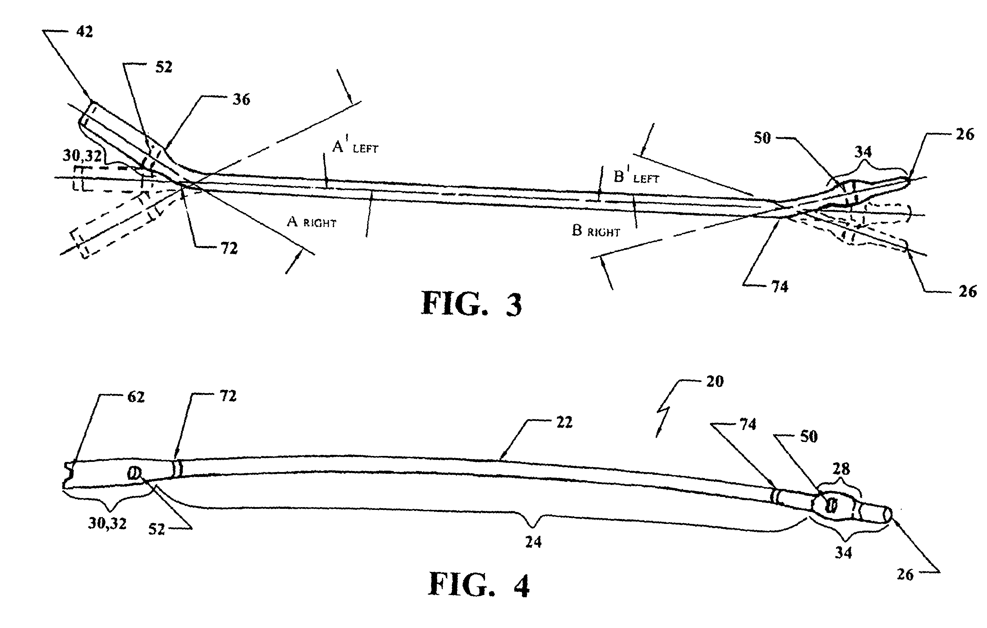

Proximal tip 42 includes attachment structure 58 for receiving a driver, described below, which may be a conventional driver used in the bone nail art. Preferably, attachment structure 58 comprises a threaded axial bore 60 extending along axis 54 and engageable with an externally threaded driver. A notch 62 extends across bore 60 and, as is known, aligns the driver for purposes of later drilling and cross fastening nail 20, as will be discussed below. As further shown in Figs. 2 and 4, nail 20 is curved in an anterior direction along a radius of curvature 70 to generally conform nail 20 to the typical femoral curvature. Preferably, this radius of curvature 70 is in the range of 30 inches to 60 inches. A proximal bend 72 and a distal bend 74 are formed, respectively, in central section 24 directly adjacent proximal and distal end sections 32, 34. These bends 72, 74 are made in the same direction, i.e., laterally to one side of nail 20 or the other as shown best in Fig. 3. The solid lines in Fig. 3 illustrate lateral bends 72, 74 out of the sagital plane and generally in the coronal plane at angles A, B for inserting nail 20 into a left femur. Angles A', B' corresponding to the respective lateral bends of proximal and distal end sections 32, 34, shown in phantom, facilitate use of nail 20 in the right femur. As a unique feature of this invention, interlocking nail 20 may be custom bent by the surgeon just prior to use, not only to facilitate insertion in the right or left femur, but also to accommodate other particular shapes necessary for a particular patient. For example, some patients may have deformities necessitating one or more corrective osteotomies or fractures made by the surgeon. These osteotomies may also be fixed using nail 20.

In the preferred embodiment, nail 20 is formed from titanium, although other materials such as those known in the art may be used as well. Central section 24 is of solid cross section and at least substantially constant diameter with a smooth outer surface to facilitate removal in 6-9 months. Proximal and distal end sections 32, 34 are also solid, except for holes 50, 52, bore 60 and notch 62. As appreciated from Fig. 2, the axes of holes 50, 52 are coplanar. In order to provide the desired flexibility of central section 24, while retaining the cross fastening feature of the invention, fastener receiving areas 28, 30 have a cross sectional dimension greater than the cross sectional dimension of central section 24. In general, central section 24 may be formed with a solid cylindrical cross section having a diameter of between 4 mm and 7 mm. Fastener receiving area 28 has a generally bulbous, rounded shape, while proximal fastener receiving area 30, which is preferably continuous with distal end section 32, has a cylindrical cross sectional shape. At the cross section taken along the axes of respective holes 50, 52, the respective ratios of the cross sectional dimensions at these locations is at least about 1.3:1 relative to the cross sectional dimension of central section 24. In exemplary embodiments of the invention, a nail 20 substantially as shown in Fig. 2, had a cross sectional dimension of 5.5 mm for central section 24, an 8.5 mm cross sectional dimension for proximal end section 32, and also a maximum 8.5 mm cross sectional dimension at the largest diameter portion of distal fastener receiving area 28. This nail was used on children generally weighing less than 100 lbs. For children weighing more than 100 lbs., the 8.5 mm dimensions at the proximal and distal fastener receiving areas remained the same, while the cross sectional dimension for central section 24 was increased slightly to 6.5 mm to provide additional strength but still provide the desirable flexure.

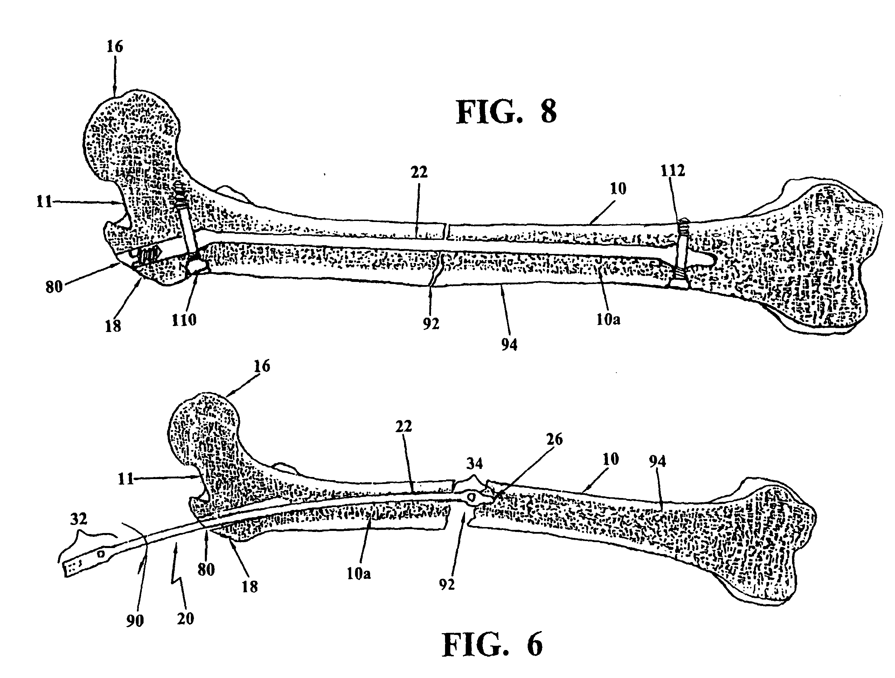

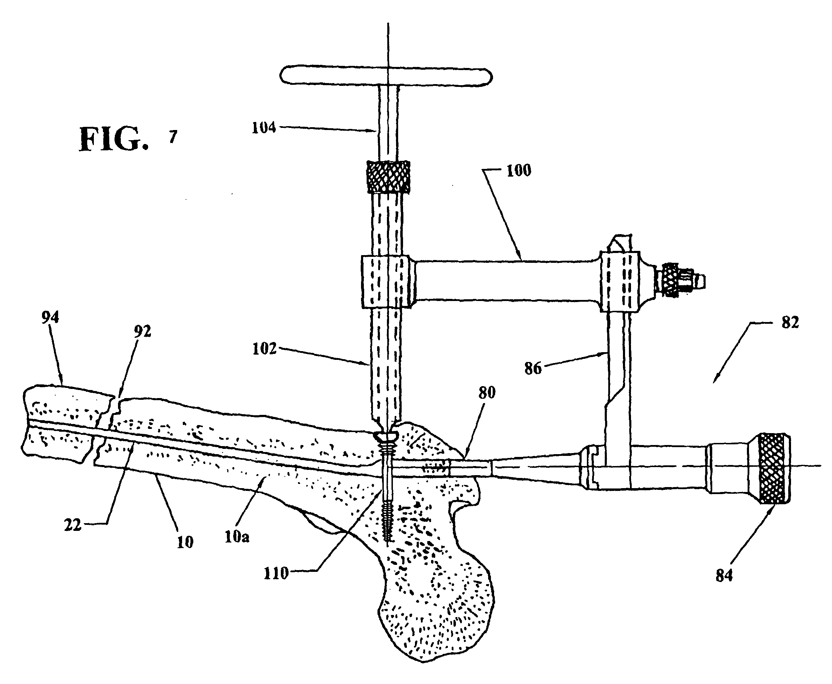

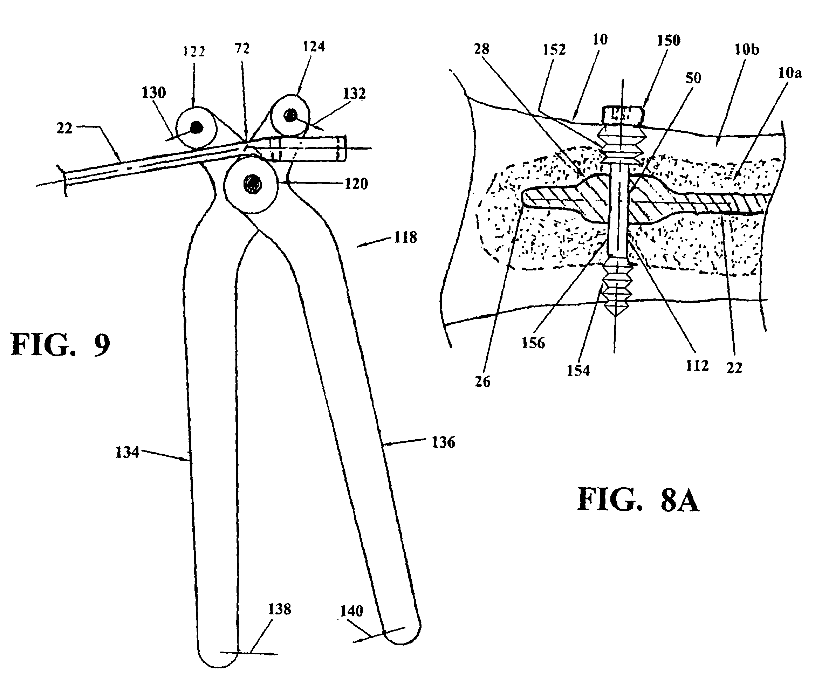

The insertion and fixation techniques according to the preferred embodiment of the invention are best illustrated in Figs. 5-8. In accordance with the invention, an insertion point 80 is created in the greater trochanter 18 of the femur 10 of a child, for example, at a distinctly lateral position relative to the piriformis fossa 11. Intramedullary canal 10a of femur 10 is drilled and reamed in a known manner to accept bone nail 20. A driver 82 is coupled with proximal tip 42, also in a known manner, and the surgeon impacts the head 84 of the driver 82 while holding the handle portion 86. Bend 74 facilitates better positioning of distal tip 26 upon insertion of nail 20 by allowing distal tip 26 to naturally follow the intramedullary canal 10a relative to the angle of the insertion point 80 in the greater trochanter 18. As shown in Fig. 6, bone nail 20 may be rotated in either direction represented by arrow 90 as the distal tip 26 approaches and crosses the fracture 92. This allows the surgeon, using fluoroscopy, to more easily locate and enter the intramedullary canal 10a of the distal bone segment 94. After the bone nail 20 is fully inserted, as shown in Fig. 7, a drill rig 100 is attached to the proximal tip 42 through securement to handle portion 86. The drill rig 100 aligns a drill guide 102 with the proximal fastener receiving hole 52 and a drill (not shown) is used to form a hole through femur 10 in line with hole 52. A screw driving mechanism 104 is then used to insert a cross fastener 110 at this location. Using conventional fluoroscopy techniques, a second hole is drilled and a second cross fastener 112, preferably of the same design as fastener 110, is inserted through the distal hole 50, as shown in Figs. 8 and 8A. More specifically, and as represented by distal fastener 112 in Fig. 8A, fastener 112 comprises a drive head 150, a proximal threaded portion 152, a distal threaded portion 154, and a central unthreaded portion 156 which is received within hole 50. Threaded portions 152, 154 are securely engaged within cortical layer 10b of femur 10. In this manner, bone nail 20 is interlocked to femur 10 at proximal and distal locations thereby preventing undesirable rotational and/or lengthwise bone movements at the fracture site. In this interlocked or fixed position, proximal tip 42 is presented directly at the insertion point 80 on the greater trochanter 18 so that, upon healing of the fracture, the cross fasteners 110, 112 may be removed and threaded bore 60 may be engaged to withdraw nail 20 from intramedullary canal 10a.

Although bends 72, 74 may be pre-made by a manufacturer of nail 20, for example, the present invention further contemplates a manual bending device as shown in Fig. 9. Using this device, nail 20 may be placed by the surgeon in a bending device 118 jaw structure comprising three rollers 120, 122, 124 with one roller 120 acting as a fulcrum and two opposite rollers 122, 124 applying forces in the direction of arrows 130, 132. When the handles 134, 136 of the device are squeezed together in the direction of arrows 138, 140, proximal end section 32 will be bent relative to central section 24 to form bend 72 as best illustrated in Fig. 3 and as previously described. The same procedure may be used by the surgeon to bend distal end section 34 just prior to insertion within intramedullary canal 10a. This aspect of the invention allows the surgeon to custom bend these or other portions of the nail 20 to suit the needs of a particular patient prior to or during surgery.

It may be appreciated by one skilled in the art that additional embodiments may be contemplated, including similarly designed nails for fixing fractures in other long bones of child and adolescent patients. It can also be contemplated that similar nails could be useful for animal long-bone fractures.

In the foregoing description, certain terms have been used for brevity, clarity, and understanding, but no unnecessary limitations are to be implied therefrom beyond the requirements of the prior art, because such words are used for description purposes herein and are intended to be broadly construed. Moreover, the embodiments of the apparatus illustrated and described herein are by way of example, and the scope of the invention is not limited to the exact details of construction.

Having now described the invention, the construction, the operation and use of preferred embodiment thereof, and the advantageous new and useful results obtained thereby, the new and useful constructions, and reasonable mechanical equivalents thereof obvious to those skilled in the art, are set forth in the appended claims.

## Claims

1. An intramedullary nail (20) for insertion within an intramedullary canal of a long bone and fixing a fracture in the long bone, the nail comprising:
an elongate member (22) having a longitudinal axis, a proximal end section (32), a distal end section (34) and a solid central section (24) extending between said proximal (32) and distal (34) end sections, said proximal (32) and distal (34) end sections respectively including proximal (28) and distal (30) fastener receiving areas, said fastener receiving areas (28, 30) each having at least one hole (50, 52) extending transverse to the longitudinal axis for receiving a cross fastener adapted to secure to the bone on opposite sides of said elongate member (22), said proximal and distal end sections (32, 34) thereby providing rigid anchoring locations relative to said central section (24) and said central section providing flexibility to promote healing of the fracture,
**characterised in that** the proximal and distal fastener receiving areas (28, 30) are of greater cross-sectional dimensions than said central section (24).

2. The intramedullary nail of claim 1, wherein said central section (24) is malleable to allow bending of at least the proximal end section (32) with respect to the central section (24).

3. The intramedullary nail recited in claim 1, wherein the central section (24) of said elongate member is curved between said fastener receiving areas (28, 30) in a sagital plane transverse to respective axes of the holes (50, 52), said curved central section (24) thereby adapted to conform with the intramedullary canal.

4. The intramedullary nail of claim 3, wherein said elongate member (22) further includes a proximal bend (72) located distally of the fastener receiving area (28) of said proximal end section (32), said proximal bend (72) forming an acute angle relative to the sagital plane containing the axis of curvature of said central section (24).

5. The intramedullary nail of claim 4, wherein said elongate member (22) further includes a distal bend (74) located proximally of the fastener receiving area (30) of said distal end section (34), said distal bend (74) forming an acute angle relative to the sagital plane containing the axis of curvature of said central section (24) and being on the same side of the sagital plane as the proximal bend (72).

6. The intramedullary nail of claim 1, wherein the ratio of the cross sectional dimensions of the respective proximal and distal fastener receiving areas (28, 30) at the axes of said holes (50, 52) relative to the cross sectional dimension of said central section (24) is at least about 1.3:1.

7. The intramedullary nail of claim 1, wherein said distal fastener receiving area (30) is tapered on proximal and distal ends thereof and said proximal fastener (28) receiving area is tapered on a distal end thereof.

8. The intramedullary nail of claim 1, wherein the holes (50, 52) extend along respective axes and the axes of said holes are generally coplanar.

9. The intramedullary nail of claim 1, further comprising cross fasteners (110, 112) respectively received in the holes (50, 52), each cross fastener having a threaded distal tip (154), a threaded proximal shank (152) and an unthreaded portion (156) between the threaded distal tip (154) and the threaded proximal shank (152), said unthreaded portion (156) adapted to be received in one of said holes (50, 52) and said threaded distal tip (154) and proximal shank (152) adapted to engage bone matter on opposite sides of said one hole (50, 52).

10. An intramedullary nailing system for fixing a fracture in a long bone of a patient haying an intramedullary canal, the system comprising:
an intramedullary nail according to any preceding claim, and
a bending device (118) having jaw structure configured to hold the elongate member (22) and bend at least one of the proximal and distal end sections (32, 34) at angle relative to said central section (24).

11. The system of claim 10, wherein said bending device (118) further comprises a pair of manually operable handles (134, 136) coupled with said jaw structure and adapted to be squeezed together to move the jaw structure.

## Patentansprüche

1. Marknagel (20) zum Einsetzen in eine Markhöhle eines langen Knochens und Fixieren einer Fraktur in dem langen Knochen, wobei der Nagel folgendes umfaßt:
ein längliches Element (22) mit einer Längsachse, einem proximalen Endabschnitt (32), einem distalen Endabschnitt (34) und einem massiven Mittelabschnitt (24), der sich zwischen dem proximalen (32) und dem distalen (34) Endabschnitt erstreckt, wobei der proximale (32) und der distale (34) Endabschnitt jeweils einen proximalen (28) und einen distalen (30) Befestigungselement-Aufnahmebereich einschließen, wobei die Befestigungselement-Aufnahmebereiche (28, 30) jeder wenigstens ein Loch (50, 52) haben, die quer zur Längsachse verlaufen, um ein zum Befestigen des Knochens an gegenüberliegenden Seiten des länglichen Elements (22) geeignetes Querbefestigungselement aufzunehmen, wobei der proximale und der distale Endabschnitt (32, 34) dadurch starre Verankerungsstellen im Verhältnis zum Mittelabschnitt (24) bereitstellen und der Mittelabschnitt Flexibilität gewährleistet, um die Heilung der Fraktur zu fördern,
**dadurch gekennzeichnet, daß** der proximale und der distale Befestigungselement-Aufnahmebereich (28, 30) größere Querschnittsabmessungen haben als der Mittelabschnitt (24).

2. Marknagel nach Anspruch 1, bei dem der Mittelabschnitt (24) schmiedbar ist, um ein Biegen wenigstens des proximalen Endabschnitts (32) im Verhältnis zum Mittelabschnitt (24) zu ermöglichen.

3. Marknagel nach Anspruch 1, bei dem der Mittelabschnitt (24) des länglichen Elements zwischen den Befestigungselement-Aufnahmebereichen (28, 30) in einer Sagittalebene quer zu jeweiligen Achsen der Löcher (50, 52) gekrümmt wird, wobei der gekrümmte Mittelabschnitt (24) dadurch geeignet ist, mit der Markhöhle übereinzustimmen.

4. Marknagel nach Anspruch 3, bei dem das längliche Element (22) außerdem eine in distaler Richtung vom Befestigungselement-Aufnahmebereich (28) des proximalen Endabschnitts (32) angeordnete proximale Biegung (72) einschließt, wobei die proximale Biegung (72) einen spitzen Winkel im Verhältnis zu der Sagittalebene bildet, welche die Krümmungsachse des Mittelabschnitts (24) enthält.

5. Marknagel nach Anspruch 4, bei dem das längliche Element (22) außerdem eine in proximaler Richtung vom Befestigungselement-Aufnahmebereich (30) des distalen Endabschnitts (34) angeordnete distale Biegung (74) einschließt, wobei die distale Biegung (74) einen spitzen Winkel im Verhältnis zu der Sagittalebene bildet, welche die Krümmungsachse des Mittelabschnitts (24) enthält, und auf der gleichen Seite der Sagittalebene liegt wie die proximale Biegung (72).

6. Marknagel nach Anspruch 1, bei dem das Verhältnis der Querschnittsabmessungen der jeweiligen proximalen und distalen Befestigungselement-Aufnahmebereiche (28, 30) an den Achsen der Löcher (50, 52) relativ zu der Querschnittsabmessung des Mittelabschnitts (24) wenigstens etwa 1,3:1 beträgt.

7. Marknagel nach Anspruch 1, bei dem der distale Befestigungselement-Aufnahmebereich (30) an dem proximalen und dem distalen Ende desselben verjüngt wird und der proximale Befestigungselement-Aufnahmebereich (28) an einem distalen Ende desselben verjüngt wird.

8. Marknagel nach Anspruch 1, bei dem die Löcher (50, 52) längs jeweiliger Achsen verlaufen und die Achsen der Löcher allgemein in der gleichen Ebene liegen.

9. Marknagel nach Anspruch 1, der außerdem Querbefestigungselemente (110, 112) umfaßt, die jeweils in den Löchern (50, 52) aufgenommen werden, wobei jedes Querbefestigungselement eine distale Gewindespitze (154), einen proximalen Gewindeschaft (152) und einen gewindelosen Abschnitt (156) zwischen der distalen Gewindespitze (154) und dem proximalen Gewindeschaft (152) hat, wobei der gewindelose Abschnitt (156) dafür geeignet ist, in einem der Löcher (50, 52) aufgenommen zu werden, und die distale Gewindespitze (154) und der proximale Schaft (152) dafür geeignet sind, Knochenmasse auf gegenüberliegenden Seiten des einen Lochs (50, 52) in Eingriff zu nehmen.

10. Marknagelungssystem zum Fixieren einer Fraktur in einem langen Knochen eines Patienten mit einer Markhöhle, wobei das System folgendes umfaßt:
einen Marknagel nach einem der vorhergehenden Ansprüche und
eine Biegevorrichtung (118) mit einer Klemmbackenstruktur, konfiguriert zum Halten des länglichen Elements (22) und Biegen wenigstens eines der proximalen und distalen Endabschnitte (32, 34) in einem Winkel im Verhältnis zum Mittelabschnitt (24).

11. System nach Anspruch 10, bei dem die Biegevorrichtung (118) außerdem ein Paar vom manuell zu bedienenden Griffen (134, 136) umfaßt, gekoppelt an die Klemmbackenstruktur und dafür geeignet, zusammengedrückt zu werden, um die Klemmbackenstruktur zu bewegen.

## Revendications

1. Clou intermédullaire (20) destiné à être inséré dans un canal intermédullaire d'un os long et à fixer une facture dans l'os long, le clou comprenant:
un élément allongé (22) comportant un axe longitudinal, une section d'extrémité proximale (32), une section d'extrémité distale (34) et une section centrale solide (24) s'étendant entre lesdites sections d'extrémité proximale (32) et distale (34), lesdites sections d'extrémité proximale (32) et distale (34) englobant respectivement des zones de réception proximale (28) et distale (30) d'un élément de fixation, lesdites zones de réception de l'élément de fixation (28, 30) comportant chacune au moins un trou (50, 52) s'étendant transversalement à l'axe longitudinal pour recevoir un élément de fixation transversal destiné à fixé sur l'os, sur les côtés opposés dudit élément allongé (22), lesdites sections d'extrémité proximale et distale (32, 34) établissant ainsi des emplacements d'ancrage rigides par rapport à ladite section centrale (24), ladite section centrale assurant une flexibilité pour stimuler la guérison de la facture,
**caractérisé en ce que** les zones de réception proximale et distale de l'élément de fixation (28, 30) ont une section transversale supérieure à celle de ladite section centrale (24).

2. Clou intermédullaire selon la revendication 1, dans lequel ladite section centrale (24) est malléable pour permettre le fléchissement d'au moins la section d'extrémité proximale (32) par rapport à la section centrale (24).

3. Clou intermédullaire selon la revendication 1, dans lequel la section centrale (24) dudit élément allongé est courbée entre lesdites zones de réception de l'élément de fixation (28, 30) dans un plan sagittal transversal aux axes respectifs des trous (50, 52), ladite section centrale courbée (24) pouvant ainsi s'adapter au canal intermédullaire.

4. Clou intermédullaire selon la revendication 3, dans lequel ledit élément allongé (22) englobe en outre un coude proximal (72) agencé de manière distale par rapport à la zone de réception de l'élément de fixation (28) de ladite section d'extrémité proximale (32), ledit coude proximal (72) formant un angle aigu par rapport au plan sagittal contenant l'axe de courbure de ladite section centrale (24).

5. Clou intermédullaire selon la revendication 4, dans lequel ledit élément allongé (22) englobe en outre un coude distal (74) agencé de manière proximale par rapport à la zone de réception de l'élément de fixation (30) de ladite section d'extrémité distale (34), ledit coude distal (74) formant un angle aigu par rapport au plan sagittal contenant l'axe de courbure de ladite section centrale (24) et étant agencé sur le même côté du plan sagittal que le coude proximal (72).

6. Clou intermédullaire selon la revendication 1, dans lequel le rapport entre les dimensions de la section transversale des zones de réception proximale et distale respectives de l'élément de fixation (28, 30) au niveau des axes desdits trous (50, 52) et la dimension de la section transversale de ladite section centrale (24) correspond au moins à 1,3:1.

7. Clou intermédullaire selon la revendication 1, dans lequel ladite zone de réception distale de l'élément de fixation (30) est effilée sur les extrémités proximale et distale correspondantes, ladite zone de réception proximale de l'élément de fixation (28) étant effilée sur une extrémité distale correspondante.

8. Clou intermédullaire selon la revendication 1, dans lequel les trous (50, 52) s'étendent le long des axes respectifs, les axes desdits trous étant en général coplanaires.

9. Clou intermédullaire selon la revendication 1, comprenant en outre des éléments de fixation transversaux (110, 112) reçus respectivement dans les trous (50, 52), chaque élément de fixation transversal comportant une pointe distale filetée (154), une tige proximale filetée (152) et une partie non filetée (156) entre la pointe distale filetée (154) et la tige proximale filetée (152), ladite partie non filetée (156) étant destinée à être reçue dans un desdits trous (50, 52), ladite pointe distale filetée (154) et la tige proximale (152) étant destinées à s'engager dans la matière osseuse sur les côtés opposés dudit un trou (50, 52).

10. Système d'enclouage intermédullaire pour fixer une facture dans un os long d'un patient comportant un canal intermédullaire, le système comprenant:
un clou intermédullaire selon l'une quelconque des revendications précédentes, et
un dispositif de flexion (118) ayant une structure à mâchoires, destiné à retenir l'élément allongé (22) et à fléchir au moins une des sections d'extrémité proximale et distale (32, 34) à un angle par rapport à ladite section centrale (24).

11. Système selon la revendication 10, dans lequel ledit dispositif de flexion (118) comprend en outre une paire de poignées à actionnement manuel (134, 136) accouplées à ladite structure à mâchoires et destinées à être rapprochées par serrage pour déplacer la structure à mâchoires.
